# EUROPÄISCHE PATENTSCHRIFT

(11) **EP 0 332 022 B1**
(45) Veröffentlichungstag und Bekanntmachung des Hinweises auf die Patenterteilung: **30.11.1994**
(21) Anmeldenummer: 89103479.5
(22) Anmeldetag: 28.02.1989
(51) Int. Cl.: C07K 3/08, C07K 17/06, C12P 21/00, C12N 1/02, G01N 33/532, G01N 33/58

(54) **Magnetische Protein-Konjugate, Verfahren zu ihrer Herstellung und ihre Verwendung**
Magnetic protein conjugates, process for their production and their use
Conjugués protéiniques magnétiques, procédé pour leur préparation et leur application

(30) Priorität: 10.03.1988 DE 3807904
(43) Veröffentlichungstag der Anmeldung: 13.09.1989
(73) Patentinhaber: BEHRINGWERKE Aktiengesellschaft, 35001 Marburg (DE)
(72) Erfinder: Hermentin, Peter, Dr., D-3550 Marburg (DE); Dönges, Reiner, D-3563 Dautphetal (DE); Enssle, Karlheinz, Dr., D-3550 Marburg (DE); Kurrle, Roland, Dr., D-3550 Marburg (DE); Seiler, Friedrich Robert, Dr., D-3550 Marburg (DE)

(56) Entgegenhaltungen:
- EP-A- 0 089 880
- EP-A- 0 125 995
- WO-A-87/02063
- JOURNAL OF PROTEIN CHEMISTRY, Band 2, Nr. 3, 1983, New York, NY (US); M.D. PARTIS et al., Seiten 263-277#
- THE LANCET, Band 1, Nr. 8368, 1984, London (GB); J.G. TRELEAVEN et al., Seiten 70-73#

## Beschreibung

Die Erfindung betrifft magnetische Protein-Konjugate der allgemeinen Formel I, 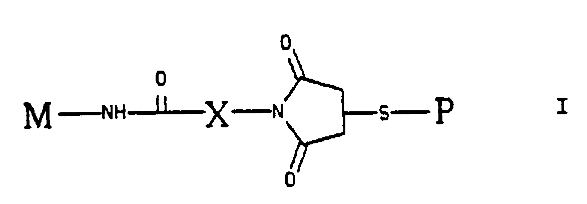
für welche gilt:
M ist ein dispergierbares, magnetisch reagierendes Material oder Partikel, welches Aminogruppen trägt, P ist ein Protein, welches eine oder mehrere Mercaptogruppen trägt, und X ist eine organisch-chemische Struktur, welche die beiden Liganden auf chemischem Wege verknüpft.

X ist bevorzugt ein aliphatischer, aromatischer, alicyclischer, alicyclisch-aliphatischer oder aromatisch-aliphatischer Spacer, welcher gegebenenfalls jeweils in geeigneter Weise substituiert sein kann, bevorzugt -(CH₂)ₙ- mit n = 1 - 8, bevorzugt mit n = 1 - 5 und besonders bevorzugt mit n = 2 oder 3 oder
X ist bevorzugt Phenylen oder substituiertes Phenylen, wobei die beiden Liganden ortho-, meta- oder para-ständig sein können, und wobei ein gegebenenfalls im Phenylen-Ring vorhandener Substituent eine Methyl-, Hydroxy-, Methoxy-, Acetoxy-, Nitro- oder Cyanogruppe oder ein Chlor- oder Bromatom sein kann oder
X ist bevorzugt Phenylen-(CH₂)ₙ- mit n = 1 - 5, bevorzugt mit n = 3, wobei die Phenylen-Gruppe mit der Succinimidyl-Gruppierung verknüpft ist oder
X ist bevorzugt -CHR-, wobei R eine Aminosäure-Seitenkette darstellt, bevorzugt die Seitenkette der Aminosäuren Alanin, Serin, Threonin oder Methionin und besonders bevorzugt die Seitenkette von Alanin oder
X ist bevorzugt Methylencyclohexyl, wobei die Methylengruppe mit der Succinimidylgruppierungverknüpft ist und bezüglich der mit dem Cyclohexyl-Ring verknüpften Carbonyl-Funktion bevorzugt in Position 4 des Cyclohexyl-Rings angeknüpft ist.

X kann auch so beschaffen sein, daß es auf chemischem oder enzymatischem Wege gespalten werden kann.

P kann ein Protein sein, in welchem die Mercaptogruppe entweder in natürlicher Weise vorhanden sind oder durch Reduktion von Disulfidbindungen erzeugt oder durch chemische Reaktion eingeführt werden.

P ist insbesondere ein Immunglobulin oder Immunglobulin-Rest, bevorzugt ein monoklonaler Antikörper oder ein Fab-, Fab'- oder F(ab')₂- Fragment, ein Antigen oder ein Enzym-, Hormon-, Lectin- oder Wachstumsfaktorrest.

P ist bevorzugt ein monoklonaler Antikörper der IgG- oder IgM-Klasse, insbesondere ein monoklonaler Antikörper, welcher gegen ein Antigen gerichtet ist, welches in wäßrigen Salzlösungen oder Körperflüssigkeiten in gelöster Form vorhanden ist oder ein monoklonaler Antikörper, welcher gegen ein Antigen gerichtet ist, welches auf Zellen exprimiert ist, wobei die das Antigen exprimierenden Zellen insbesondere Zellen des myeloischen oder lymphatischen Systems, Zellen des peripheren Bluts, insbesondere B-Lymphozyten, T- Lymphozyten oder deren Vorläuferzellen oder Tumorzellen, insbesondere Tumorzellen des Knochenmarks sein können. Solche Zellen können auch Erythrozyten, Bakterien, Mykoplasmen oder Protozoen sein. Im Rahmen der Erfindung sollen aber auch Viren als Zellen verstanden werden.

M ist bevorzugt ein dispergierbares Partikel mit Metalloxid- Core und einem Aminogruppen tragenden Hüllmantel, wobei das Metalloxid-Core eine Gruppe paramagnetischer Substanzen einschließen kann, bevorzugt ein Partikel dessen Durchmesser zwischen etwa 0,1 µ und etwa 100 µ, bevorzugt jedoch zwischen etwa 0,1 µ und etwa 1,5 µ liegt.

Die Erfindung betrifft ferner ein Verfahren zur Herstellung eines magnetischen Protein-Konjugats der allgemeinen Formel I sowie die Verwendung eines Konjugats der Formel I zur spezifischen Entfernung von Zellen oder löslichen Antigenen, Rezeptoren, Substraten, Co-Faktoren oder Kohlenhydrat-Determinanten aus wäßrigen Salzlösungen oder Körperflüssigkeiten sowie die Verwendung im Rahmen eines Diagnostikums bzw. als Diagnostikum sowie insbesondere die Verwendung zur Knochenmarks-Depletion oder zur HLA-Typisierung.

Eine Knochenmarkstransplantation ist unter anderem bei der Behandlung bestimmter Formen der Leukämie und der Panmyelopathie (Knochenmarksschwund) oftmals die einzige Möglichkeit der Therapie.

Bei Leukämien und gewissen lymphoiden Neoplasien werden die Patienten bisweilen einer Ganzkörperbestrahlung mit extrem hoher Dosis und/oder einer aggressiven Chemotherapie unterworfen. Bei einer derartigen Behandlung werden die normalen Stammzellen des Knochenmarks, die Vorläufer aller Blutzellen, vollständig zerstört. Dem Patienten wird daher Knochenmark eines geeigneten Spenders reinfundiert, von dem sich Zellen in den Knochenmarkräumen des Empfängers ansiedeln und so die Neuentwicklung des Blutbildungs- und Immunsystems ermöglichen. Dieses Verfahren nennt man allogene Knochenmarkstransplantation.

Verantwortlich für das hohe Risiko der allogenen Knochenmarkstransplantation sind unter anderem die dem Patienten im reinfundierten Knochenmark mitübertragenen T-Lymphozyten des Spenders, welche die Zellen des Empfängers als fremd erkennen und sie deshalb angreifen und zerstören. Diese für den Patienten oft lebensbedrohende Knochenmarksunverträglichkeit nennt man "graft-versus-host"-Reaktion oder "graft-versus-host"-Erkrankung (GVHD). Die Risiken dieser "graft-versus-host"-Erkrankung können zum einen dadurch vermindert werden, daß man dem Patienten nach Möglichkeit genau typisiertes Knochenmark besonders geeigneter Spender, meist aus dem Verwandtenkreis, reinfundiert. Zum anderen können sie aber auch dadurch reduziert werden, daß man unerwünschte Zellpopulationen, wie sie z. B. T-Lymphozyten des Spender-Knochenmarks darstellen können, vor der Reinfusion in den Patienten selektiv eliminiert. Diese Elimination von Spender-T-Zellen kann z. B. durch selektive Lysis der zu entfernenden Zellen in Gegenwart von Komplement oder durch selektive Abtötung der T-Zellen mit Hilfe sogenannter Immuntoxine oder durch ein anderes Verfahren, z. B. durch magnetische Zell-Depletion des Knochenmarks erfolgen.

Eine derartige Zell-Depletion von Knochenmark kann man auf relativ einfache Weise so durchführen, daß man das Knochenmark mit einem monoklonalen Antikörper von der Maus inkubiert, welcher z. B. spezifisch gegen die T-Zellen des Knochenmarks gerichtet ist und sich infolgedessen nur an die T-Zellen bindet. Derartige, mit monoklonalen Antikörpern von der Maus belegte T-Zellen können nun in einem zweiten Schritt dadurch entfernt werden, daß man sie z. B. mit anti-Maus-Immunglobulin vom Kaninchen, welches an magnetische Partikel gebunden ist, inkubiert, wodurch die T-Lymphozyten in spezifischer Weise mit dem magnetischen Material belegt werden, so daß sie sich mit Hilfe eines Magneten aus dem Knochenmark entfernen lassen (s. hierzu Vartdal et al., Transplantation (1987), 43, 366-371 und die dort zitierte Literatur).

In analoger Weise lassen sich aus dem Knochenmark auch andere Zellpopulationen, etwa Tumorzellen, entfernen, was für die sogenannte autologe Knochenmarkstransplantation von Bedeutung ist (s. hierzu Kvalheim et al., Cancer Research (1987), 47, 846-851 und die dort zitierte Literatur). Dabei kann, wie von Kvalheim et al., ibid., beschrieben, der die Tumorzellen erkennende monoklonale Antikörper auch direkt an die magnetischen Partikel gebunden werden, so daß der oben erwähnte Zweitantikörper (Kaninchen anti-Maus) nicht mehr benötigt wird.

Das vorstehend beschriebene Verfahren zur Knochenmarks-Depletion mit Hilfe monoklonaler oder polyklonaler Antikörper, welche an magnetische Partikel gebunden sind, ist noch sehr jung und bedarf der weiteren Entwicklung und Erprobung. Hierfür geeignete magnetische Partikel stehen heute in verschiedenster Form käuflich zur Verfügung, und ihre Herstellung wurde mehrfach in der Patentliteratur beschrieben (s. z. B. Chagnon et al., EP 0125995 A2 (Priorität US 493991 vom 12.05.83), Advanced Magnetics, oder Ughelstad et al., WO 8303920 vom 10.11.83, SINTEF). Von diesen magnetischen Partikeln ist bekannt, daß sie aus einem Metalloxid-Core bestehen, in welchem paramagnetische Substanzen eingeschlossen sein können, und daß das Core von einem Hüllmantel umgeben ist, welcher reaktive Gruppen, wie z. B. Aminophenyl-, Amino-, Carboxyl-, Hydroxy- oder Sulfhydrylgruppen, tragen kann, welche zur Kopplung von Proteinen verwendet werden können (Chagnon et al., EP 0125995 A2).

Bekannt ist beispielsweise, daß Carboxylgruppen tragende Partikel in Gegenwart eines Kondensationsmittels mit Aminogruppen von Proteinen zur Reaktion gebracht werden können (Chagnon et al., EP 0125995 A2).

Bekannt ist ferner die Kopplung von Proteinen an Aminogruppen tragende magnetische Partikel unter Verwendung von Glutaraldehyd, wobei die Kopplung jeweils über die Aminogruppen erfolgt (Chagnon et al., EP 0125995 A2).

Bekannt ist weiterhin, daß sich Hydroxygruppen tragende Partikel durch Reaktion mit p-Toluolsulfonylchlorid aktivieren lassen, und daß solchermaßen aktivierte Partikel mit Aminogruppen von Proteinen zur Reaktion gebracht werden können (Kvalheim et al., Cancer Research (1987), 47, 846-851).

All diese Kopplungsverfahren haben gemeinsam, daß das Protein jeweils über seine freien Aminogruppen an die Partikel angeknüpft wird. Eine derartige Kopplung über Aminogruppen kann jedoch bei monoklonalen Antikörpern von erheblichem Nachteil sein, weil hierbei bisweilen die Spezifität und Reaktivität der Antikörper beeinträchtigt wird. Dies ist eine Folge der Tatsache, daß sich die Aminogruppen bei einem Antikörper sozusagen statistisch über das ganze Molekül verteilen und daher auch in der Antigen-Bindungsstelle der Fab-Fragmente lokalisiert sind, was bei einer Kopplung über diese Aminogruppen einen Spezifitätsverlust bewirkt.

Es ist weiterhin bekannt (The Lancet, Band 1, Nr. 8368, 1984, Seiten 70-73, Treleaven et al.), daß sich Antikörper auch ohne chemische Verknüpfung rein adsorptiv auf magnetische Partikel aufziehen lassen, wenn die Partikel durch ein Styrol-Divinylbenzol-Copolymerisat umhüllt sind, da sich Protein bekanntlich unspezifisch an Polystyrol anbindet.

Auch bei diesem Verfahren muß jedoch mit einer Beeinträchtigung der Antikörperspezifität und -reaktivität gerechnet werden. Ein weiterer gravierender Nachteil dieses Verfahrens besteht jedoch darin, daß sich adsorptiv gebundene Antikörper bei der Knochenmarks-Depletion wieder ablösen und so dem Patienten bei der Reinfusion des depletierten Knochenmarks mit verabreicht werden können, was insbesondere bei einem vorangegangenen Therapieversuch mit monoklonalen Antikörpern zu ernsten Nebenreaktionen führen könnte. Dieses Problem ist jedoch bekannt und soll durch kovalente Anknüpfung der Antikörper an die magnetischen Partikel überwunden werden.

Bekannt ist auch, daß magnetische Partikel mit Polystyrol-Hülle den gravierenden Nachteil haben, daß sie zu Aggregation neigen und sich an Zellen auch unspezifisch anlagern.

Ausgehend von diesem Stand der Technik ist es die Aufgabe der vorliegenden Erfindung, eine Methode zu entwickeln, bei welcher monoklonale Antikörper an magnetische Partikel a) kovalent und b) nicht über ihre Aminogruppen gekoppelt werden. Aufgabe der vorliegenden Erfindung ist es daher, mit anderen Worten, ein Kopplungsverfahren zu finden, bei welchem die Antigen-Bindungsstelle des Antikörpers nicht verändert wird bzw. die Kopplung des Antikörpers abseits der Antigen-Bindungsstelle erfolgt.

Gelöst wird diese erfindungsgemäße Aufgabe durch Herstellung magnetischer Protein-Konjugate der allgemeinen Formel I von Seite 1.

Es wurde nun gefunden, daß sich magnetische Partikel, welche als reaktive Gruppen freie Aminogruppen tragen, auf einfache Weise in magnetische Partikel überführen lassen, welche als reaktive Gruppen Maleimido-Funktionen tragen. Derartige Partikel sind neu.

Weiterhin wurde gefunden, daß magnetische Partikel, welche Maleimido-Funktionen tragen, problemlos mit Proteinen konjugiert werden können, welche Mercapto gruppen besitzen, wobei die Mercapto gruppen im Protein entweder bereits nativ vorhanden oder auf chemischem Wege eingeführt oder durch Reduktion vorhandener Disulfidbindungen erzeugt werden können.

Insbesondere wurde gefunden, daß magnetische Partikel, welche Maleimido-Funktionen tragen, problemlos mit monoklonalen Antikörpern konjugiert werden können, wenn man die Interketten-Disulfid-Bindungen der Antikörper durch selektive Reduktion in freie SH-Gruppen überführt, welche mit den Maleimido-Funktionen der magnetischen Partikel unter Ausbildung einer stabilen Thioetherbindung zur Reaktion gebracht werden können. Diese Art der Kopplung monoklonaler Antikörper an magnetische Partikel ist ebenfalls neu.

Überraschenderweise wurde gefunden, daß die Spezifität und Reaktivität der über Thioether-Bindungen an magnetische Partikel gekoppelte Antikörper voll erhalten bleibt, weil durch die Kopplung des Antikörpers über seine Hinge-Region seine Antigen- Bindungsstelle nicht verändert oder beeinträchtigt wird. Hierin liegt ein besonderer Vorteil der Erfindung gegenüber den bislang bekannten Kopplungsverfahren, bei denen die Antikörper, wie vorstehend beschrieben, entweder rein adsorptiv oder über Reaktion ihrer Aminogruppen auf magnetische Partikel aufgezogen werden, was sowohl die Spezifität als auch die Reaktivität der konjugierten Antikörper beeinträchtigen kann. Gegenüber der adsorptiven Kopplung besteht überdies bei der vorliegenden Erfindung der Vorteil, daß die Antikörper an die magnetischen Partikel chemisch gebunden sind und sich infolgedessen bei der Verwendung erfindungsgemäßer magnetischer Antikörper-Konjugate, z. B. zur Knochenmarks-Depletion, nicht von den Partikeln ablösen.

Es wurde weiterhin gefunden, daß sich die erfindungsgemäßen magnetischen Antikörper-Konjugate, z. B. bei der Depletion von Knochenmark, wegen ihrer hohen Spezifität als besonders vorteilhaft erweisen.

Weiterhin wurde gefunden, daß sich die erfindungsgemäßen magnetischen Antikörper-Konjugate auch im Rahmen eines diagnostischen Verfahrens bzw. als Diagnostikum, insbesondere z. B. bei der HLA-Typisierung, wegen ihrer hohen Spezifität als vorteilhaft erweisen.

Die Herstellung erfindungsgemäßer magnetischer Antikörper-Konjugate ist nachfolgend für verschiedene monoklonale Antikörper, welche gegen Zellen des Knochenmarks gerichtet sind, beispielhaft beschrieben; jedoch engen die genannten Beispiele die Erfindung nicht ein. Weiterhin ist die Verwendung der beispielhaft hergestellten magnetischen Antikörper-Konjugate zur Depletion von Zellen des Knochenmarks sowie zur HLA-Typisierung ebenfalls beispielhaft beschrieben, ohne die Verwendung auf die genannten Beispiele zu beschränken.

### Verfahren zur Herstellung eines magnetischen Protein-Konjugats der allgemeinen Formel I

Aminogruppen tragende magnetische Partikel M werden in einem geeigneten Lösungsmittel mit einem mit Aminogruppen reagierenden Maleimido-Spacer der allgemeinen Formel II, 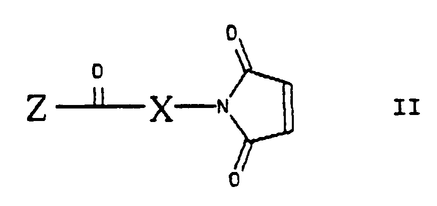
worin Z eine geeignete reaktive Abgangsgruppe ist, unter Ausbildung einer Amidbindung zu einer Verbindung der allgemeinen Formel III 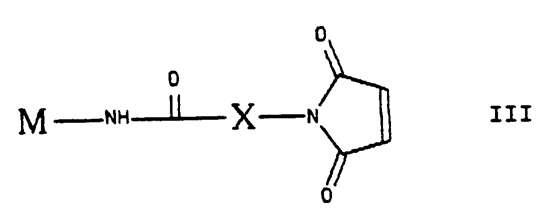
umgesetzt, welche schließlich in einem geeigneten wäßrigen salzhaltigen Lösungsmittel, welches Proteine nicht denaturiert, wie z. B. physiologische Kochsalzlösung oder eine mit Phosphat gepufferte Kochsalzlösung, mit einem Mercaptogruppen tragenden Protein P zu einer Verbindung der allgemeinen Formel I umgesetzt wird.

Geeignete Lösungsmittel zur Kopplung einer Verbindung der Formel II an magnetische Partikel müssen so beschaffen sein, daß die jeweils zur Kopplung verwendeten magnetischen Partikel in ihren physikalischen und magnetischen Eigenschaften, insbesondere in ihrer Größe, Dispergierbarkeit und Oberflächenbeschaffenheit, durch das verwendete Lösungsmittel nicht beeinträchtigt werden. Für magnetische Partikel, wie sie z. B. in EP 0125995 A2 oder WO 8303920 beschrieben sind, wurde als geeignetes Lösungsmittel z. B. eine Mischung aus Wasser und Dimethylformamid aufgefunden, wobei der Substituent Z der Formel II z. B. und bevorzugt Succinimidyloxy ist. Ein geeignetes Lösungsmittel kann aber z. B. auch wasserfreies Aceton sein, wobei dann der Substituent Z der Formel II neben Succinimidyloxy auch ein Halogen, wie z. B. Chlor oder Brom, oder ein Pseudohalogenid, wie z. B. Cyanid, oder eine Acylgruppe, wie z. B. Acetyl oder p-Nitrobenzoyl, oder eine andere reaktive Abgangsgruppe, wie z. B. eine Tosylgruppe, sein kann, wobei in all den Fällen, in denen Z nicht Succinimidyloxy ist, die Reaktion bevorzugterweise in Gegenwart einer geeigneten Base, wie z. B. Natriumcarbonat, durchgeführt wird.

### Verfahren zur Depletion von Zellen

Eine Suspension eines zu depletierenden Zellgemischs in einer salzhaltigen, bevorzugt physiologischen wäßrigen Lösung oder in einer Körperflüssigkeit wird mit einer Verbindung der Formel I bei einer geeigneten Temperatur zwischen z. B. 0° C und 40° C, bevorzugt unter Schütteln, ebenfalls bevorzugt unter sterilen Bedingungen, über einen geeigneten Zeitraum inkubiert, und danach werden die magnetischen Partikel durch einen geeigneten Magneten aus der Lösung abgetrennt.

Geeignete Temperaturen sind beispielsweise 0° C, Raumtemperatur oder 37° C, bevorzugt jedoch Raumtemperatur. Die Zeitdauer der Inkubation richtet sich jeweils nach der verwendeten Inkubationstemperatur und nach der Bindungsreaktivität des Antikörpers und kann z. B. von wenigen Minuten bis zu z. B. 2 Stunden betragen. Bevorzugterweise wird z. B. bei Raumtemperatur z. B. über einen Zeitraum von 10 bis 20 Minuten inkubiert.

### Verfahren zur Isolierung löslicher bioorganischer Moleküle

Dieses Verfahren folgt im wesentlichen dem Verfahren zur Depletion von Zellen.

### Beispiele:

Die nachfolgenden Beispiele dienen der näheren Erläuterung der Erfindung, schränken die Erfindung jedoch nicht ein.

Magnetische Partikel, welche in der beschriebenen Weise mit monoklonalen Antikörpern zur Reaktion gebracht wurden, werden nachfolgend als "Magnetobeads" bezeichnet, wobei ihre Spezifität jeweils durch Voranstellung der jeweiligen Antikörper-Bezeichnung wiedergegeben wird.

### Beispiel 1:

### Konjugation magnetischer Partikel gemäß EP 0125995 A2 mit Gamma-maleimidobutyrat-Spacer

0,5 ml einer käuflichen Suspension der magnetischen Partikel (BioMag M4100, Sebak^{R}) wurden 3 x mit Phosphat-gepufferter Kochsalzlösung pH 7.2 (PBS) gewaschen und in 6 ml PBS resuspendiert. Diese Suspension wurde mit einer frisch hergestellten Lösung von 20 mg N-(Gamma-maleimidobutyryloxy)succinimid (GMBS, Calbiochem) in 4 ml trockenem Dimethylformamid versetzt und 1 h bei Raumtemperatur geschüttelt. Dann wurden die Partikel bei 3000 x g abzentrifugiert, 3 x mit je 20 ml PBS gewaschen und in 6 ml PBS resuspendiert.
*) Bem.: Alle verwendeten monoklonalen Antikörper waren in einer Konzentration von 1 mg/ml in einer Lösung von 0,1 mol/l Natriumcitrat-Puffer pH 6.6 und 50 g/l Saccharose lyophilisiert.

### Beispiel 2:

### Allgemeine Vorschrift zur Kopplung monoklonaler Antikörper* an Maleimidogruppen tragende magnetische Partikel gemäß Beispiel 1

1 mg lyophilisierter monoklonaler Antikörper wurde in 0,5 ml Wasser gelöst, mit 2 mg Dithiothreitol versetzt und 30 min bei Raumtemperatur inkubiert. Der reduzierte Antikörper wurde mittels Gelfiltration über Sephadex® G25 in isotonischer Kochsalzlösung pH 7.2 in einem Elutionsvolumen von 4 ml isoliert und zu der gemäß Beispiel 1 hergestellten Suspension magnetischer Partikel zugesetzt. Die Mischung (10 ml) wurde 45 min unter Schütteln bei Raumtemperatur inkubiert. Dann wurden die Partikel bei 3500 x g abzentrifugiert, 3 x mit je 20 ml PBS gewaschen, in 10 ml PBS pH 7.2 resuspendiert und bei 4° C aufbewahrt.

Gegebenenfalls wurde die Suspension mit 0,2 % Natriumazid oder mit 0,2 % Natriumazid + 0,1 % BSA (bovine serum albumin) + 0,2 % Tween 20 versetzt.

Die nach Beispiel 2 gekoppelten monoklonalen Antikörper sind in Tabelle 1 zusammengefaßt.

**Tabelle 1**

| Gemäß Beispiel 2 hergestellte Magnetobeads | | |
|---|---|---|
| monoklonaler Antikörper | Klasse und Isotyp | Spezifität |
| BMA 0110 | IgG2b | CD2 |
| BMA 0111 | IgG1 | CD2 |
| BMA 030 | IgG2a | CD3 |
| BMA 033 | IgG3 | CD3 |
| BMA 031 | IgG2b | TCR |
| BMA 041 | IgM | CD4 |
| BMA 0117 | IgG2a | CD7 |
| BMA 081 | IgG2a | CD8 |
| VIL-A1 | IgM | CD10 |
| SV393 | IgG1 | Beta-2-M |
| BMA 022 | IgG2a | HLA-DR |
| BMA 0210 | IgM | Monozyten |
| 84-24/91-8 | IgG1 | TSH |

### Beispiel 3:

### Positive Reaktion von BMA 041-Magnetobeads mit T-Zellen

40 µl einer gemäß Beispiel 2 hergestellten Suspension von BMA 041-Magnetobeads in PBS wurden mit 120 µl einer T-Zellsuspension (Klon 5/87-7-A3; CD4⁺, CD8⁻, CD2⁺, CD3⁺, T-Zellrezeptor-positiv; 1 x 10⁴ Zellen/µl in RPMI 1640) 10 min unter Schütteln bei Raumtemperatur inkubiert und unverzüglich in einer Zählkammer mikroskopisch betrachtet und abfotografiert. Die Zellen waren mit den magnetischen Partikeln rosettiert bzw. verklumpt.

### Beispiel 4:

### Negative Reaktion von BMA 041-Magnetobeads mit B-Zellen

40 µl einer gemäß Beispiel 2 hergestellten Suspension von BMA 041-Magnetobeads in PBS wurden mit 120 µl einer B-Zellsuspension (B-Zellinie GR, 1 x 10⁴ Zellen/µl in RPMI 1640) wie in Beispiel 3 inkubiert und danach mikroskopisch betrachtet und abfotografiert. Eine Rosettierung bzw. Verklumpung wurde nicht beobachtet.

### Beispiel 5:

### Positive Reaktion von BMA 081-Magnetobeads mit T-Zellen

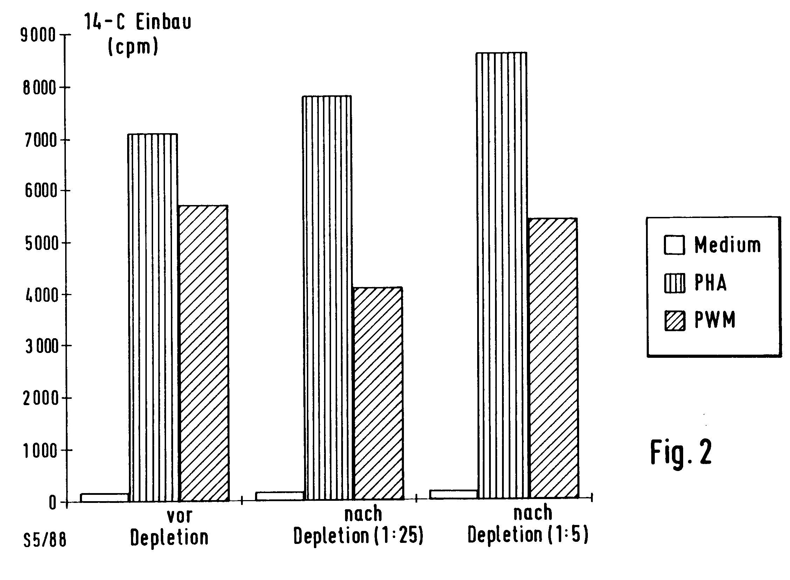

40 µl einer gemäß Beispiel 2 hergestellten Suspension von BMA 081-Magnetobeads wurden wie in Beispiel 3 mit T-Zellen (Klon 5/87-4-B6; CD8⁺, CD4⁻, CD2⁺, CD3⁺, T-Zellrezeptorpositiv) inkubiert und danach mikroskopisch betrachtet und abfotografiert (Ergebnis s. Abbildung 2a). Als Negativkontrolle dienten analog Beispiel 4 B-Zellen (B-Zellinie GR).

Die T-Zellrezeptor-positiven Zellen waren mit den magnetischen Partikeln rosettiert bzw. verklumpt. Bei der Negativkontrolle wurde eine Rosettierung bzw. Verklumpung nicht beobachtet.

### Beispiel 6:

### Positive Reaktion von BMA 031-Magnetobeads mit T-Zellen

40 µl einer gemäß Beispiel 2 hergestellten Suspension von BMA 031-Magnetobeads wurden analog Beispiel 3 mit T-Zellen (Klon 5/87-4-B6; CD8⁺, CD4⁻, CD2⁺, CD3⁺, T-Zellrezeptorpositiv) inkubiert und danach mikroskopisch betrachtet und abfotografiert. Als Negativkontrolle dienten analog Beispiel 4 B-Zellen (B-Zellinie GR). Die Antigen-positiven Zellen waren wiederum mit den magnetischen Partikeln rosettiert bzw. verklumpt; die Antigen-negativen Zellen lagen frei.

### Beispiel 7:

### Depletion von Zellpopulationen aus mononukleären Zellen

Mononukleäre Zellen (MNZ) wurden in an sich bekannter Weise (Boyum, Scand. J. Immunol. (1976), Suppl. 5, 9 - 15) über einen Ficoll®-Gradienten aus frisch gespendetem Humanblut isoliert.

Für die Depletion wurden 3 x 10⁷ MNZ in 2 ml PBS mit 1 % BSA ((w/v), Seromed) in Plastik-Röhrchen (Falcon, Nr. 2051) mit 1 ml einer Suspension von 2 mg/ml Magnetobeads in PBS gemischt und 15 min bei Raumtemperatur unter dauerndem Schütteln inkubiert. Dann wurden die Magnetobeads und die daran gebundenen Zellen mit Hilfe eines Permanentmagneten abgetrennt. Die in Suspension verbliebenen Zellen wurden bei 400xg pelletiert und in einem geeigneten Medium, z. B. PBS oder RPMI 1640, resuspendiert. Die Depletionseffizienz wurde mittels indirekter Immunfluoreszenz im Cytofluorograph(Ortho) bestimmt.

Hierzu wurden 1 x 10⁶ Zellen vor und nach Depletion einer bestimmten Zellpopulation mit 1 - 10 µg/ml Erstantikörper (einem monoklonalen Antikörper jeweils geeigneter Spezifität) und danach mit 10 - 50 µg/ml Zweitantikörper (Kaninchen-anti-Maus-Immunglobulin, F(ab)₂-Fragment, FITC-markiert, Behringwerke) in an sich bekannter Weise markiert und im Cytofluorograph ausgewertet, wobei jeweils Depletionseffizienzen von über 95 % bestimmt wurden.

Die nach Beispiel 7 depletierten Zellen sind in Tabelle 2 aufgelistet.

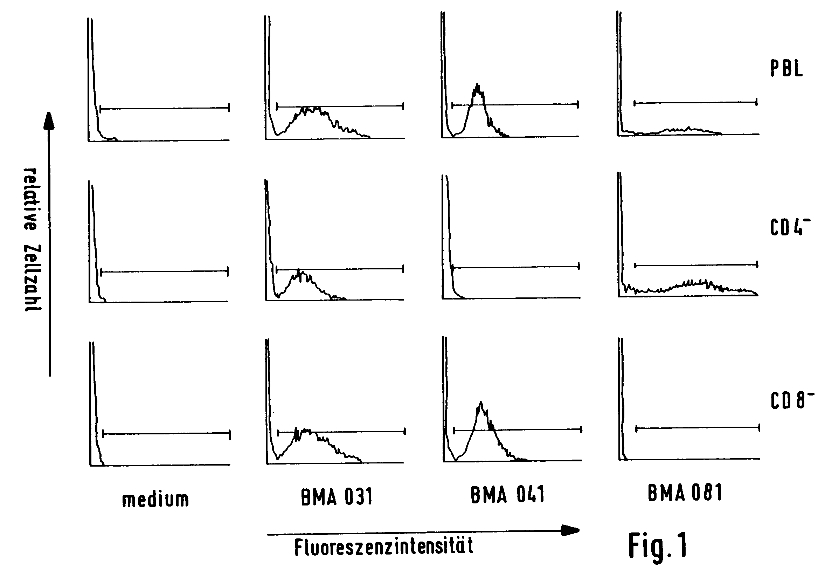

Die erzielten Ergebnisse sind für die Depletion mit BMA 041 (anti-CD4, Behringwerke AG)-Magnetobeads und BMA 081 (anti-CD8, Behringwerke AG)-Magnetobeads in Abbildung 1 beispielhaft wiedergegeben. Dabei wurde die Depletionseffizienz durch Markierung der jeweils ungebundenen Zellpopulation mit BMA 031 (anti-T-Zell-Rezeptor, Behringwerke AG), BMA 041 oder BMA 081 bestimmt. Nach Inkubation mit FITC-markiertem Zweitantikörper (s. oben) wurde die jeweilige Fluoreszenzintensität im Cytofluorograph bei linearer Verstärkung ausgewertet.

**Tabelle 2**

| Depletion von Zellen mittels gemäß Beispiel 2 hergestellter Magnetobeads | | |
|---|---|---|
| gekoppelter monoklonaler Antikörper | erkanntes Antigen (Spezifität) | depletierte Zellpopulationen |
| BMA 0110 | CD 2 | CD2⁺-T-Zellen |
| BMA 031 | TCR | TCR⁺-T-Zellen |
| BMA 041 | CD 4 | CD4⁺-T-Zellen |
| BMA 0117 | CD 7 | CD7⁺-T-Zellen |
| BMA 081 | CD 8 | CD8⁺-T-Zellen |
| VIL-A1 | CD 10 | CD10⁺-B-Zellen |

### Beispiel 8:

### Nachweis der Proliferation von T-Zellen in Kultur nach Inkubation mit ungekoppelten magnetischen Partikeln (BioMag M4100, Sebak^{R})

Mononukleäre Zellen wurden analog Beispiel 7 aus humanem Blut isoliert und mit einer Suspension ungekoppelter magnetischer Partikel (BioMag M4100, Sebak^{R}) analog Beispiel 7 inkubiert. Danach wurden die magnetischen Partikel mit Hilfe eines Permanentmagneten abgetrennt. Die verbliebenen MNZ wurden in 96-Well-Flachboden-Titerplatten (Nunc) bei einer Konzentration von jeweils 1 x 10⁵ MNZ je Well in serumfreiem Kulturmedium (Iscove, Behring-Modifikation) in Gegenwart von Pokeweed-Mitogen (PWM, Gibco ; 1 : 3000, Endverdünnung) oder von 10 µg/ml Phytohämagglutinin (PHA, Wellcome) 64 Stunden im CO₂-Brutschrank bei 37° C inkubiert. Nach 48 Stunden Kulturdauer wurden die Kulturen jeweils mit 2,7 x 10³ Bq ¹⁴C-Thymidin (Amersham) versetzt. Nach weiteren 14 Stunden Kulturdauer wurden die Zellen über einen Zell-Harvester (Innotec) auf Glasfaser-Filterplättchen abgesaugt. Die Radioaktivität auf den Filtern wurde nach Zugabe von Szintillationsflüssigkeit in einem β-Counter (Packard) bestimmt.

Abbildung 2 zeigt die Auswertung eines solchen Experiments und belegt, daß die Stimulation von MNZ mit PHA oder PWM durch eine Depletion mit ungekoppelten magnetischen Partikeln (BioMag M4100, Sebak^{R}) nicht beeinträchtigt wird.

### Beispiel 9:

### Nachweis der Proliferation von T-Zellen in Kultur in Gegenwart ungekoppelter magnetischer Partikel (BioMag M4100, Sebak^{R})

MNZ wurden unter Zusatz verschiedener Mengen ungekoppelter magnetischer Partikel (BioMag M4100, Sebak^{R}) in 96-Well-Platten verteilt und mit PHA (10 µg/ml), BMA 030 (Behringwerke, mitogen für T-Lymphozyten) (0,01 µg/ml) oder Staphylococcus aureus-Capsid (SAC, Behringwerke, mitogen für B-Lymphozyten, Endverdünnung 1 : 3000) in Analogie zu Beispiel 8 kultiviert.

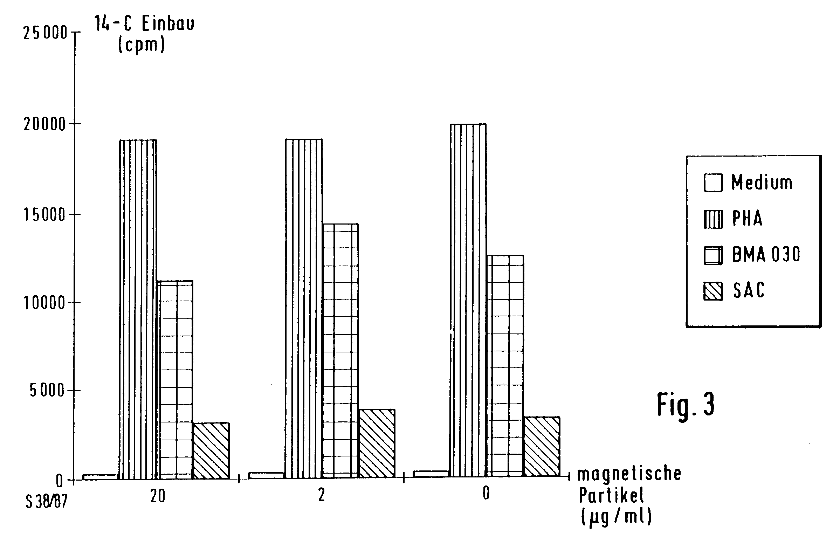

Aus Abbildung 3 ist ersichtlich, daß magnetische Partikel (BioMag M4100, Sebak^{R}) bei einer Konzentration von 2 - 20 µg/ml keinen negativen Effekt auf die Proliferation von B- und T-Zellen haben.

### Legende zu den Abbildungen

- Abb. 1:: Depletion von T-Zell-Subpopulationen mit BMA 041- und BMA 081-Magnetobeads
- PBL:: ursprüngliche Zellpopulation
- CD4⁻:: Population nach Depletion mit BMA 041-Magnetobeads
- CD8⁻:: Population nach Depletion mit BMA 081-Magnetobeads
- Abb. 2:: Proliferation von T-Zellen in Kultur nach Inkubation mit ungekoppelten magnetischen Partikeln (BioMag M4100, Sebak^{R}) und Stimulation mit Phytohämagglutinin (PHA) oder Pokeweed-Mitogen (PWM) im Vergleich zur Medium-Kontrolle
- Abb. 3:: Proliferation von T-Zellen in Kultur in Gegenwart ungekoppelter magnetischer Partikel (BioMag M4100, Sebak^{R}) nach Stimulation mit Phytohämagglutinin (PHA), anti-CD3 monoklonalem Antikörper BMA 030, oder Staphylococcus aureus-Capsid (SAC) im Vergleich zur Medium-Kontrolle

## Patentansprüche (Patentansprüche für folgende(n) Vertragsstaat(en): AT, BE, CH, DE, FR, GB, IT, LI, LU, NL, SE)

1. Magnetisches Protein-Konjugat der allgemeinen Formel I, 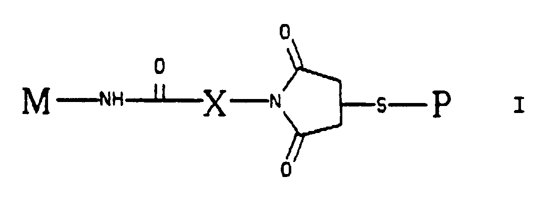 worin M ein dispergierbares, magnetisch reagierendes Material oder Partikel ist, welches Aminogruppen trägt, P ein Protein ist, welches eine oder mehrere Mercaptogruppen trägt und X eine organisch-chemische Struktur darstellt, welche die beiden Liganden auf chemischem Wege verknüpft.

2. Magnetisches Protein-Konjugat nach Anspruch 1,
**dadurch gekennzeichnet**,
daß X ein aliphatischer, aromatischer, alicyclischer, alicyclisch-aliphatischer oder aromatisch-aliphatischer Spacer ist, welcher gegebenenfalls jeweils in geeigneter Weise substituiert sein kann.

3. Magnetisches Protein-Konjugat nach Anspruch 2,
**dadurch gekennzeichnet**,
daß X -(CH₂)ₙ- mit n = 1 - 8, bevorzugt mit n = 1 - 5 und besonders bevorzugt mit n = 2 oder 3 ist.

4. Magnetisches Protein-Konjugat nach Anspruch 2,
**dadurch gekennzeichnet**,
daß X Phenylen ist, wobei die beiden Liganden ortho-, meta- oder para-ständig sein können.

5. Magnetisches Protein-Konjugat nach Anspruch 2,
**dadurch gekennzeichnet**,
daß X Phenylen-(CH₂)ₙ- mit n= 1 - 5, bevorzugt mit n = 3 ist, wobei die Phenylen-Gruppe mit der Succinimidyl-Gruppierung verknüpft ist.

6. Magnetisches Protein-Konjugat nach Anspruch 2,
**dadurch gekennzeichnet**,
daß X -CHR- ist, wobei R eine Aminosäure-Seitenkette darstellt, bevorzugt die Seitenkette der Aminosäuren Alanin, Serin, Threonin oder Methionin und besonders bevorzugt die Seitenkette von Alanin.

7. Magnetisches Protein-Konjugat nach Anspruch 2,
**dadurch gekennzeichnet**,
daß X Methylencyclohexyl ist, wobei die Methylengruppe mit der Succinimidyl - Gruppierung verknüpft ist und bezüglich der mit dem Cyclohexyl-Ring verknüpften Carbonyl-Funktion bevorzugt in Position 4 des Cyclohexyl-Rings angeknüpft ist.

8. Magnetisches Protein-Konjugat nach Anspruch 2,
**dadurch gekennzeichnet**,
daß X wie in Anspruch 4 ist, der Phenylen-Ring jedoch durch eine Methyl-, Hydroxy-, Methoxy-, Acetoxy-, Nitro- oder Cyanogruppe oder durch ein Chlor- oder Bromatom substituiert ist.

9. Magnetisches Protein-Konjugate nach Anspruch 1,
**dadurch gekennzeichnet**, daß
die Mercaptogruppen des Proteins P entweder in natürlicher Weise vorhanden sind oder durch Reduktion von Disulfidbindungen erzeugt oder durch chemische Reaktion in das Protein eingeführt werden.

10. Magnetisches Protein-Konjugate nach Anspruch 1,
**dadurch gekennzeichnet**,
daß P ein Immunglobulin oder Immunglobulin-Rest, bevorzugt ein monoklonaler Antikörper oder ein Fab-, Fab'- oder F(ab')₂-Fragment, ein Antigen oder ein Enzym-, Hormon-, Lectin- oder Wachstumsfaktorrest sein kann.

11. Magnetisches Protein-Konjugat nach Anspruch 10,
**dadurch gekennzeichnet**,
daß P einen monoklonalen Antikörper der IgG- oder IgM-Klasse darstellt.

12. Magnetisches Protein-Konjugat nach Anspruch 10,
**dadurch gekennzeichnet**,
daß P einen monoklonalen Antikörper darstellt, welcher gegen ein Antigen gerichtet ist, welches in wäßrigen Salzlösungen oder Körperflüssigkeiten in gelöster Form vorhanden ist.

13. Magnetisches Protein-Konjugat nach Anspruch 10,
**dadurch gekennzeichnet**,
daß P einen monoklonalen Antikörper darstellt, welcher gegen ein Antigen gerichtet ist, welches auf Zellen, insbesondere auf Zellen des myeloischen oder lymphatischen Systems oder des peripheren Bluts, insbesondere auf B-Lymphozyten, T-Lymphozyten oder deren Vorläuferzellen oder auf Tumorzellen, insbesondere auf Tumorzellen des Knochenmarks, exprimiert ist.

14. Magnetisches Protein-Konjugat nach Anspruch 13,
**dadurch gekennzeichnet**,
daß P einen monoklonalen Antikörper darstellt, welcher gegen ein Antigen gerichtet ist, welches auf Bakterien, Mykoplasmen oder Protozoen oder aber auf Viren exprimiert ist.

15. Magnetisches Protein-Konjugat nach Anspruch 1,
**dadurch gekennzeichnet**,
daß P ein Antigen darstellt.

16. Magnetisches Protein-Konjugat nach Anspruch 1,
**dadurch gekennzeichnet**,
daß M ein dispergierbares Partikel mit Metalloxid-Core und einem Aminogruppen tragenden Hüllmantel ist, wobei das Metalloxid-Core eine Gruppe paramagnetischer Substanzen einschließen kann.

17. Magnetisches Protein-Konjugat nach Anspruch 16,
**dadurch gekennzeichnet**,
daß der Durchmesser der Partikel zwischen etwa 0,1 µ und etwa 100 µ, bevorzugt jedoch zwischen etwa 0,1 µ und etwa 1,5 µ liegt.

18. Magnetisches Protein-Konjugat nach Anspruch 1,
**dadurch gekennzeichnet**,
daß X auf chemischem oder enzymatischem Wege gespalten werden kann.

19. Verfahren zur Herstellung eines magnetischen Protein-Konjugats der Formel I, 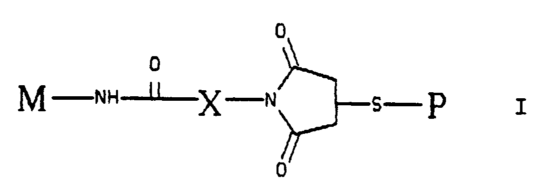 **dadurch gekennzeichnet**,
daß man Aminogruppen tragende magnetische Partikel M mit einem mit Aminogruppen reagierenden Maleimido-Spacer der allgemeinen Formel II, 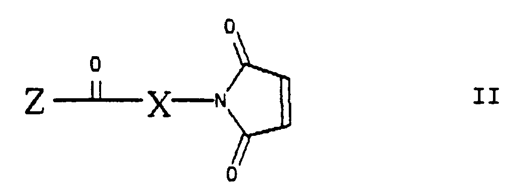 wobei Z eine reaktive Abgangsgruppe ist, unter Ausbildung einer Amidbindung zu einer Verbindung der allgemeinen Formel III 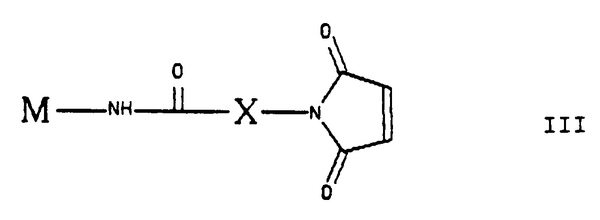 umsetzt, welche schließlich mit einem Mercaptogruppen tragenden Protein P zu einer Verbindung der allgemeinen Formel I umgesetzt wird.

20. Verfahren zur Entfernung eines gelösten Antigens, Antikörpers, Rezeptors, Substrats, Co-Faktors oder einer Kohlenhydrat-Determinante aus wäßrigen Salzlösungen oder Körperflüssigkeiten,
**dadurch gekennzeichnet**,
daß man die Lösung mit einem geeigneten magnetischen Protein-Konjugat der Formel I 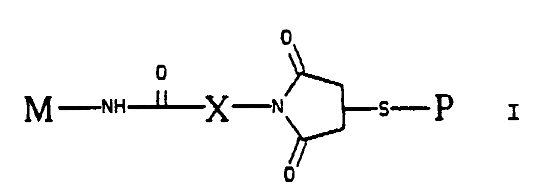 inkubiert und das magnetische Protein-Konjugat nach spezifischer Adsorption der zu entfernenden Komponente auf magnetischem Wege abtrennt und die spezifisch adsorbierte Komponente gegebenenfalls wieder vom magnetischen Protein-Konjugat abeluiert.

21. Verfahren zur Entfernung von Zellen aus wäßrigen Salzlösungen oder Körperflüssigkeiten,
**dadurch gekennzeichnet**,
daß man die Zellsuspension mit einem geeigneten magnetischen Protein-Konjugat der Formel I 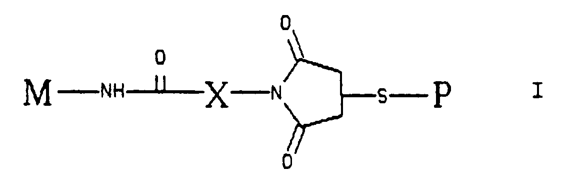 inkubiert und das magnetische Protein-Konjugat nach spezifischer Adsorption der zu entfernenden Zellen auf magnetischem Wege abtrennt und die spezifisch adsorbierten Zellen oder Partikel gegebenenfalls wieder vom magnetischen Protein-Konjugat ablöst.

22. Verwendung eines magnetischen Protein-Konjugats nach Anspruch 1 zur spezifischen Entfernung von Zellen oder löslichen Antigenen, Rezeptoren, Substraten, Co-Faktoren oder Kohlenhydrat-Determinanten aus wäßrigen Salzlösungen oder Körperflüssigkeiten oder Verwendung im Rahmen eines diagnostischen Verfahrens bzw. als Diagnostikum.

23. Verwendung eines magnetischen Protein-Konjugats nach Anspruch 22 zur Entfernung von Zellen gemäß Anspruch 13 oder 14, bevorzugt zur Knochenmarks-Depletion, oder zur HLA-Typisierung.

## Patentansprüche (Patentansprüche für folgende(n) Vertragsstaat(en): ES, GR)

1. Verfahren zur Herstellung eines magnetischen Protein-Konjugats der allgemeinen Formel I, 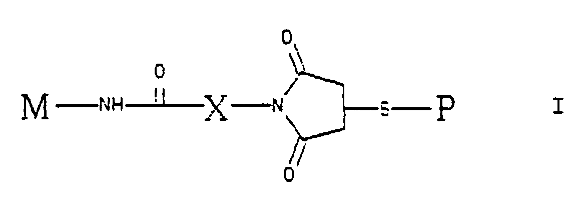 worin M ein dispergierbares, magnetisch reagierendes Material oder Partikel ist, welches Aminogruppen trägt, P ein Protein ist, welches eine oder mehrere Thiolgruppen trägt und X eine inerte organisch-chemische Struktur darstellt, welche die beiden Liganden auf chemischem Wege verknüpft,
**dadurch gekennzeichnet**,
daß man Aminogruppen tragende magnetische Partikel M mit einem mit Aminogruppen reagierenden Maleimido-Spacer der allgemeinen Formel II, 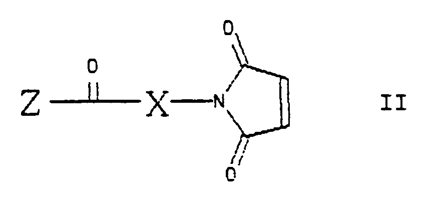 wobei Z eine reaktive Abgangsgruppe ist, unter Ausbildung einer Amidbindung zu einer Verbindung der allgemeinen Formel III 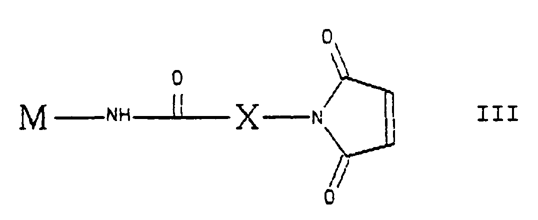 umsetzt, welche schließlich mit einem Thiolgruppen tragenden Protein P zu einer Verbindung der allgemeinen Formel I umgesetzt wird.

2. Verfahren zur Herstellung eines magnetischen Protein-Konjugats nach Anspruch 1,
**dadurch gekennzeichnet**,
daß X ein aliphatischer, aromatischer, alicyclischer, alicyclisch-aliphatischer oder aromatisch-aliphatischer Spacer ist, welcher gegebenenfalls jeweils in geeigneter Weise substituiert sein kann.

3. Verfahren zur Herstellung eines magnetischen Protein-Konjugats nach Anspruch 2,
**dadurch gekennzeichnet**,
daß X -(CH₂)ₙ- mit n = 1 - 8, bevorzugt mit n = 1 - 5 und besonders bevorzugt mit n = 2 oder 3 ist oder daß X Phenylen ist, wobei die beiden Liganden ortho-, meta- oder para-ständig sein können oder daß X Phenylen-(CH₂)ₙ- mit n= 1 - 5, bevorzugt mit n = 3 ist, wobei die Phenylen-Gruppe mit der Succinimid-Gruppierung verknüpft ist oder daß X -CHR- ist, wobei R eine Aminosäure-Seitenkette darstellt, bevorzugt die Seitenkette der Aminosäuren Alanin, Serin, Threonin oder Methionin und besonders bevorzugt die Seitenkette von Alanin oder daß X Cyclohexylmethylen ist, wobei die Methylengruppe mit der Succinimid-Gruppierung verknüpft ist und bezüglich der mit dem Cyclohexyl-Ring verknüpften Carbonyl-Funktion bevorzugt in Position 4 des Cyclohexyl- Rings angeknüpft ist oder daß X Phenylen ist, wobei der Phenylen-Ring jedoch durch eine Methyl-, Hydroxy-, Methoxy-, Acetoxy-, Nitro- oder Cyanogruppe oder durch ein Chlor- oder Bromatom substituiert ist.

4. Verfahren zur Herstellung eines magnetischen Protein-Konjugats nach Anspruch 1,
**dadurch gekennzeichnet**,
daß die Thiolgruppen des Proteins P entweder in natürlicher Weise vorhanden sind oder durch Reduktion von Disulfidbindungen erzeugt oder durch chemische Reaktion in das Protein eingeführt werden.

5. Verfahren zur Herstellung eines magnetischen Protein-Konjugats nach Anspruch 1,
**dadurch gekennzeichnet**,
daß P ein Immunglobulin oder Immunglobulin-Rest, bevorzugt ein monoklonaler Antikörper oder ein Fab-, Fab'- oder F(ab')₂-Fragment, ein Antigen oder ein Enzym-, Hormon-, Lectin- oder Wachstumsfaktorrest sein kann.

6. Verfahren zur Herstellung eines magnetischen Protein-Konjugats nach Anspruch 5,
**dadurch gekennzeichnet**,
daß P ein monoklonaler Antikörper der IgG- oder IgM-Klasse ist, bevorzugt ein monoklonaler Antikörper, welcher gegen ein Antigen gerichtet ist, welches in wäßrigen Salzlösungen oder Körperflüssigkeiten in gelöster Form vorhanden oder auf Zellen, insbesondere auf Zellen des myeloischen oder lymphatischen Systems oder des peripheren Bluts, insbesondere auf B-Lymphozyten, T-Lymphozyten oder deren Vorläuferzellen oder auf Tumorzellen, insbesondere auf Tumorzellen des Knochenmarks oder auf Bakterien, Mykoplasmen, Protozoen oder Viren exprimiert ist.

7. Verfahren zur Herstellung eines magnetischen Protein-Konjugats nach Anspruch 1,
**dadurch gekennzeichnet**,
daß P ein Antigen darstellt.

8. Verfahren zur Herstellung eines magnetischen Protein-Konjugats nach Anspruch 1,
**dadurch gekennzeichnet**,
daß M ein dispergierbares Partikel mit Metalloxid-Core und einem Aminogruppen tragenden Hüllmantel ist, wobei das Metalloxid-Core eine Gruppe paramagnetischer Substanzen einschließen kann, wobei ein Partikeldurchmesser zwischen etwa 0,1 µ und etwa 100 µ bevorzugt und ein Partikeldurchmesser zwischen etwa 0,1 µ und etwa 1,5 µ besonders bevorzugt ist.

9. Verfahren zur Herstellung eines magnetischen Protein-Konjugats nach Anspruch 1,
**dadurch gekennzeichnet**,
daß X auf chemischem oder enzymatischem Wege gespalten werden kann.

10. Verfahren zur Entfernung eines gelösten Antigens, Antikörpers, Rezeptors, Substrats, Co-Faktors oder einer Kohlenhydrat-Determinante aus wäßrigen Salzlösungen oder Körperflüssigkeiten,
**dadurch gekennzeichnet**,
daß man die Lösung mit einem geeigneten magnetischen Protein-Konjugat der Formel I 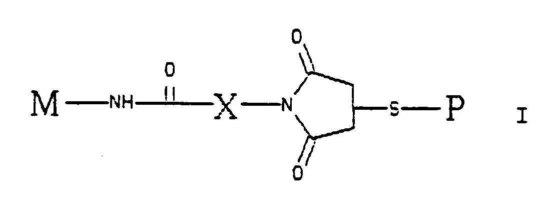 inkubiert und das magnetische Protein-Konjugat nach spezifischer Adsorption der zu entfernenden Komponente auf magnetischem Wege abtrennt und die spezifisch adsorbierte Komponente gegebenenfalls wieder vom magnetischen Protein-Konjugat abeluiert.

11. Verfahren zur Entfernung von Zellen aus wäßrigen Salzlösungen oder Körperflüssigkeiten,
**dadurch gekennzeichnet**,
daß man die Zellsuspension mit einem geeigneten magnetischen Protein-Konjugat der Formel I 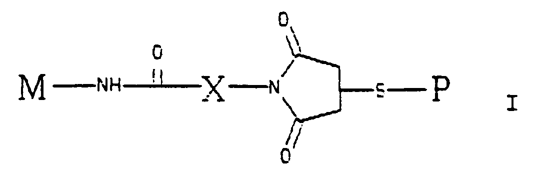 inkubiert und das magnetische Protein-Konjugat nach spezifischer Adsorption der zu entfernenden Zellen auf magnetischem Wege abtrennt und die spezifisch adsorbierten Zellen oder Partikel gegebenenfalls wieder vom magnetischen Protein-Konjugat ablöst.

12. Verwendung eines magnetischen Protein-Konjugats nach Anspruch 1 zur spezifischen Entfernung von Zellen oder löslichen Antigenen, Rezeptoren, Substraten, Co-Faktoren oder Kohlenhydrat-Determinanten aus wäßrigen Salzlösungen oder Körperflüssigkeiten oder Verwendung im Rahmen eines diagnostischen Verfahrens bzw. als Diagnostikum.

13. Verwendung eines magnetischen Protein-Konjugats nach Anspruch 12 zur Entfernung von Zellen gemäß Anspruch 11, bevorzugt zur Knochenmarks-Depletion, oder zur HLA-Typisierung.

## Claims (Claims for the following Contracting State(s): AT, BE, CH, DE, FR, GB, IT, LI, LU, NL, SE)

1. A magnetic protein conjugate of the formula I, 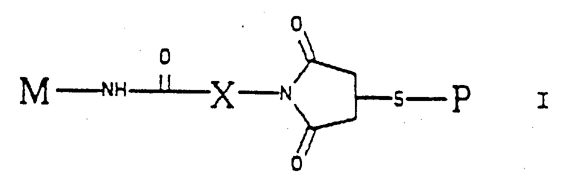 wherein M is a dispersible, magnetically reactive material or particle which carries amino groups, P is a protein which carries one or more mercapto groups, and X is an organochemical structure which chemically links the two ligands.

2. A magnetic protein conjugate as claimed in claim 1, wherein X is an aliphatic, aromatic, alicyclic, alicyclic-aliphatic or aromatic-aliphatic spacer which can in each case be optionally substituted in a suitable manner.

3. A magnetic protein conjugate as claimed in claim 2, wherein X is -(CH₂)ₙ- with n = 1 - 8, preferably with n = 1 - 5, and particularly preferably with n = 2 or 3.

4. A magnetic protein conjugate as claimed in claim 2, wherein X is phenylene, it being possible for the two ligands to be in the ortho, meta or para positions.

5. A magnetic protein conjugate as claimed in claim 2, wherein X is phenylene-(CH₂)ₙ- with n = 1 - 5, preferably with n = 3, the phenylene group being linked to the succinimidyl grouping.

6. A magnetic protein conjugate as claimed in claim 2, wherein X is -CHR-, where R is an amino acid side chain, preferably the side chain of the amino acids alanine, serine, threonine or methionine, and particularly preferably the side chain of alanine.

7. A magnetic protein conjugate as claimed in claim 2, wherein X is methylenecyclohexyl, where the methylene group is linked to the succinimidyl grouping and, relative to the carbonyl function linked to the cyclohexyl ring, is preferably connected in position 4 of the cyclohexyl ring.

8. A magnetic protein conjugate as claimed in claim 2, wherein X is as in claim 4, with, however, the phenylene ring being substituted by a methyl, hydroxyl, methoxy, acetoxy, nitro or cyano group, or by a chlorine or bromine atom.

9. A magnetic protein conjugate as claimed in claim 1, wherein the mercapto groups of the protein P are either present in a natural manner or produced by reducing disulfide bonds or introduced into the protein by chemical reaction.

10. A magnetic protein conjugate as claimed in claim 1, wherein P can be an immunoglobulin or immunoglobulin residue, preferably a monoclonal antibody or a Fab, Fab' or F(ab')₂ fragment, an antigen or an enzyme residue, hormone residue, lectin residue or growth factor residue.

11. A magnetic protein conjugate as claimed in claim 10, wherein P is a monoclonal antibody of the IgG or IgM class.

12. A magnetic protein conjugate as claimed in claim 10, wherein P is a monoclonal antibody which is directed against an antigen which is present in dissolved form in aqueous salt solutions or body fluids.

13. A magnetic protein conjugate as claimed in claim 10, wherein P is a monoclonal antibody which is directed against an antigen which is expressed on cells, in particular on cells of the myeloid or lymphatic system or of the peripheral blood, in particular on B lymphocytes, T lymphocytes or their precursor cells, or on tumor cells, in particular on tumor cells of the bone marrow.

14. A magnetic protein conjugate as claimed in claim 13, wherein P is a monoclonal antibody which is directed against an antigen which is expressed on bacteria, mycoplasmas or protozoa or else on viruses.

15. A magnetic protein conjugate as claimed in claim 1, wherein P is an antigen.

16. A magnetic protein conjugate as claimed in claim 1, wherein M is a dispersible particle having a metal oxide core and an encasing sheath carrying amino groups, where the metal oxide core can include a group of paramagnetic substances.

17. A magnetic protein conjugate as claimed in claim 16, wherein the diameter of the particles is between about 0.1 µ and about 100 µ, preferably, however, between about 0.1 µ and about 1.5 µ.

18. A magnetic protein conjugate as claimed in claim 1, wherein X can be cleaved chemically or enzymically.

19. A process for preparing a magnetic protein conjugate of the formula I, 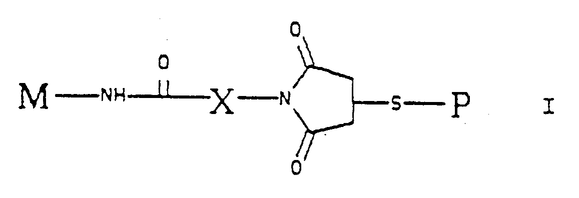 wherein
magnetic particles M carrying amino groups are reacted with a maleimido spacer, which reacts with amino groups, of the formula II, 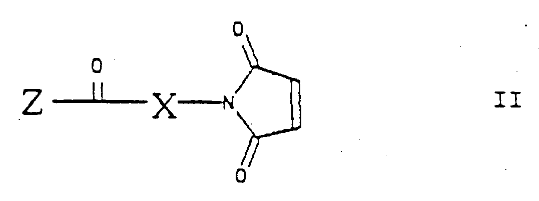 in which Z is a reactive leaving group, with the formation of an amide bond to yield a compound of the formula III, 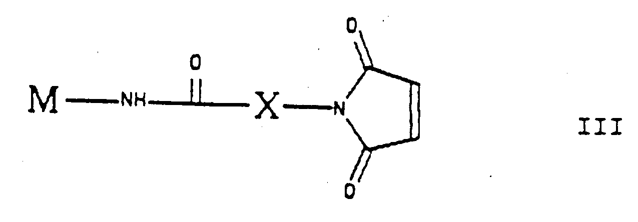 which is finally reacted with a protein P carrying mercapto groups to yield a compound of the formula I.

20. A process for removing a dissolved antigen, antibody, receptor, substrate or cofactor, or a carbohydrate determinant, from aqueous salt solutions or body fluids, wherein the solution is incubated with a suitable magnetic protein conjugate of the formula I 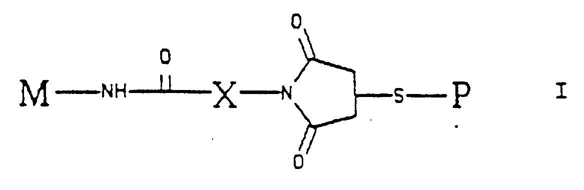 and the magnetic protein conjugate is separated off magnetically, after specifically adsorbing the component to be removed, and the specifically adsorbed component is, where appropriate, eluted off the magnetic protein conjugate again.

21. A process for removing cells from aqueous salt solutions or body fluids, wherein the cell suspension is incubated with a suitable magnetic protein conjugate of the formula I 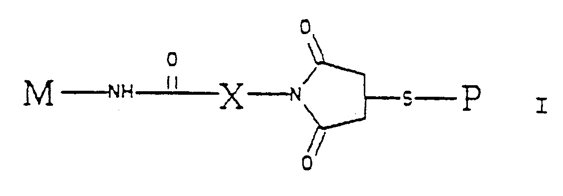 and the magnetic protein conjugate is separated off magnetically, after specifically adsorbing the cells to be removed, and the specifically adsorbed cells or particles are, where appropriate, detached again from the magnetic protein conjugate.

22. The use of a magnetic protein conjugate as claimed in claim 1 for specifically removing cells or soluble antigens, receptors, substrates, cofactors or carbohydrate determinants from aqueous salt solutions or body fluids, or the use within the framework of a diagnostic process or as a diagnostic agent.

23. The use of a magnetic protein conjugate as claimed in claim 22 for removing cells as claimed in claim 13 or 14, preferably for bone marrow depletion or for HLA typing.

## Claims (Claims for the following Contracting State(s): ES, GR)

1. A process for preparing a magnetic protein conjugate of the formula I, 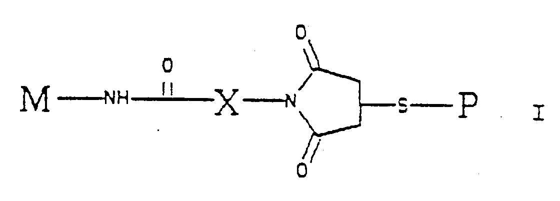 in which M is a dispersible, magnetically reactive material or particle which carries amino groups, P is a protein which carries one or more thiol groups, and X is an inert organochemical structure which chemically links the two ligands, wherein magnetic particles M carrying amino groups are reacted with a maleimido spacer, which reacts with amino groups, of the formula II, 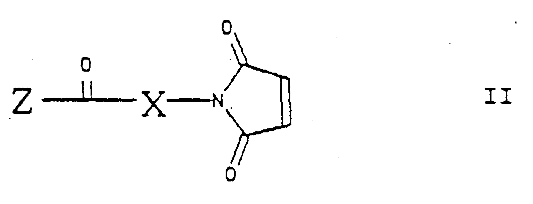 in which Z is a reactive leaving group, with the formation of an amide bond to yield a compound of the formula III, 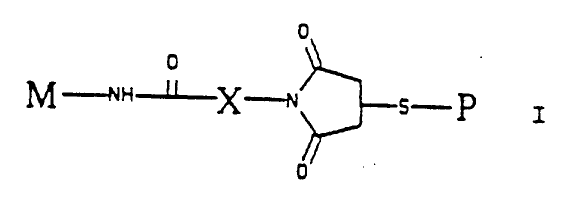 which is finally reacted with a protein P carrying thiol groups to yield a compound of the formula I.

2. The process for preparing a magnetic protein conjugate as claimed in claim 1, wherein X is an aliphatic, aromatic, alicyclic, alicyclic-aliphatic or aromatic-aliphatic spacer which can in each case be optionally substituted in a suitable manner.

3. The process for preparing a magnetic protein conjugate as claimed in claim 2, wherein X is -(CH₂)ₙ- with n = 1 - 8, preferably with n = 1 - 5, and particularly preferably with n = 2 or 3, or wherein X is phenylene, it being possible for the two ligands to be in the ortho, meta or para positions, or wherein X is phenylene-(CH₂)ₙ- with n = 1 - 5, preferably with n = 3, the phenylene group being linked to the succinimide grouping, or wherein X is -CHR-, where R is an amino acid side chain, preferably the side chain of the amino acids alanine, serine, threonine or methionine, and particularly preferably the side chain of alanine, or wherein X is cyclohexylmethylene, where the methylene group is linked to the succinimide grouping and, relative to the carbonyl function linked to the cyclohexyl ring, is preferably connected in position 4 of the cyclohexyl ring, or wherein X is phenylene, the phenylene ring being substituted, however, by a methyl, hydroxyl, methoxy, acetoxy, nitro or cyano group, or by a chlorine or bromine atom.

4. The process for preparing a magnetic protein conjugate as claimed in claim 1, wherein the thiol groups of the protein P are either present in a natural manner or produced by reducing disulfide bonds or introduced into the protein by chemical reaction.

5. The process for preparing a magnetic protein conjugate as claimed in claim 1, wherein P can be an immunoglobulin or immunoglobulin residue, preferably a monoclonal antibody or a Fab, Fab' or F(ab')₂ fragment, an antigen or an enzyme residue, hormone residue, lectin residue or growth factor residue.

6. The process for preparing a magnetic protein conjugate as claimed in claim 5, wherein P is a monoclonal antibody of the IgG or IgM class, preferably a monoclonal antibody which is directed against an antigen which is present in dissolved form in aqueous salt solutions or body fluids, or is expressed on cells, in particular on cells of the myeloid or lymphatic system or of the peripheral blood, in particular on B lymphocytes, T lymphocytes or their precursor cells, or on tumor cells, in particular on tumor cells of the bone marrow, or on bacteria, mycoplasmas, protozoa or viruses.

7. The process for preparing a magnetic protein conjugate as claimed in claim 1, wherein P is an antigen.

8. The process for preparing a magnetic protein conjugate as claimed in claim 1, wherein M is a dispersible particle having a metal oxide core and an encasing sheath carrying amino groups, where the metal oxide core can include a group of paramagnetic substances, a particle diameter being preferred between about 0.1 µ and about 100 µ, and a particle diameter being particularly preferred between about 0.1 µ and about 1.5 µ.

9. The process for preparing a magnetic protein conjugate as claimed in claim 1, wherein X can be cleaved chemically or enzymically.

10. A process for removing a dissolved antigen, antibody, receptor, substrate or cofactor, or a carbohydrate determinant, from aqueous salt solutions or body fluids, wherein the solution is incubated with a suitable magnetic protein conjugate of the formula I 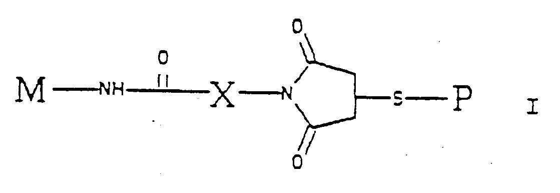 and the magnetic protein conjugate is separated off magnetically, after specifically adsorbing the component to be removed, and the specifically adsorbed component is, where appropriate, eluted off the magnetic protein conjugate again.

11. A process for removing cells from aqueous salt solutions or body fluids, wherein the cell suspension is incubated with a suitable magnetic protein conjugate of the formula I 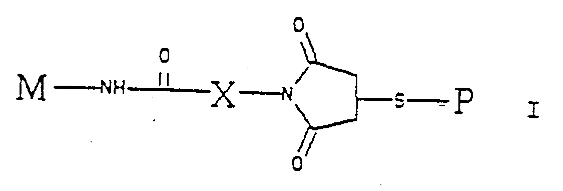 and the magnetic protein conjugate is separated off magnetically, after specifically adsorbing the cells to be removed, and the specifically adsorbed cells or particles are, where appropriate, detached again from the magnetic protein conjugate.

12. The use of a magnetic protein conjugate as claimed in claim 1 for specifically removing cells or soluble antigens, receptors, substrates, cofactors or carbohydrate determinants from aqueous salt solutions or body fluids, or the use within the framework of a diagnostic process or as a diagnostic agent.

13. The use of a magnetic protein conjugate as claimed in claim 12 for removing cells as claimed in claim 11, preferably for bone marrow depletion or for HLA typing.

## Revendications (Revendications pour l'(les) Etat(s) contractant(s) suivant(s): AT, BE, CH, DE, FR, GB, IT, LI, LU, NL, SE)

1. Conjugué protéinique magnétique de formule générale I 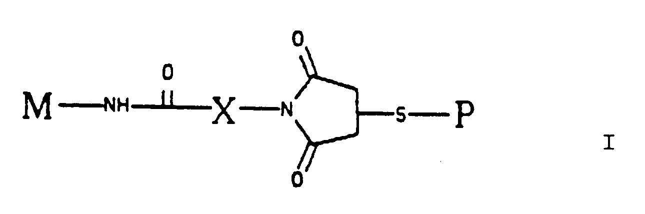 dans laquelle M est un matériau ou une particule dispersible, à réaction magnétique, qui porte des groupes amino, P est une protéine qui porte un ou plusieurs groupes mercapto, et X représente une structure chimique organique qui relie par voie chimique les deux ligands.

2. Conjugué protéinique magnétique selon la revendication 1, caractérisé en ce que X est un espaceur aliphatique, aromatique, alicyclique, alicyclique-aliphatique ou aromatique-aliphatique, qui peut éventuellement être dans chaque cas substitué de façon appropriée.

3. Conjugué protéinique magnétique selon la revendication 2, caractérisé en ce que X est un groupe -(CH₂)ₙ- dans lequel n = 1 à 8, de préférence dans lequel n = 1 à 5 et en particulier dans lequel n = 2 ou 3.

4. Conjugué protéinique magnétique selon la revendication 2, caractérisé en ce que X est le groupe phénylène, les deux ligands pouvant être en position ortho, méta ou para.

5. Conjugué protéinique magnétique selon la revendication 2, caractérisé en ce que X est un groupe phénylène-(CH₂)ₙ- dans lequel n = 1 à 5, de préférence dans lequel n = 3, le groupe phénylène étant relié au groupement succinimidyle.

6. Conjugué protéinique magnétique selon la revendication 2, caractérisé en ce que X est -CHR-, R représentant une chaîne latérale d'un aminoacide, de préférence la chaîne latérale de l'aminoacide alanine, sérine, thréonine ou méthionine, et en particulier la chaîne latérale de l'alanine.

7. Conjugué protéinique magnétique selon la revendication 2, caractérisé en ce que X est le groupe méthylènecyclohexyle, le groupe méthylène étant relié au groupement succinimidyle, et, en ce qui concerne la fonction carbonyle reliée au cycle cyclohexyle, celle-ci étant de préférence liée en position 4 du cycle cyclohexyle.

8. Conjugué protéinique magnétique selon la revendication 2, caractérisé en ce que X est tel que défini dans la revendication 4, mais le cycle phénylène est substitué par un groupe méthyle, hydroxy, méthoxy, acétoxy, nitro ou cyano, ou par un atome de chlore ou de brome.

9. Conjugué protéinique magnétique selon la revendication 1, caractérisé en ce que les groupes mercapto de la protéine P soit sont présents de façon naturelle, soit engendrés par réduction de liaisons disulfure, ou sont introduits dans la protéine par une réaction chimique.

10. Conjugué protéinique magnétique selon la revendication 1, caractérisé en ce que P peut être une immunoglobuline ou un reste d'immunoglobuline, de préférence un anticorps monoclonal ou un fragment Fab, Fab' ou F(ab')₂, un antigène ou un reste d'enzyme, d'hormone, de lectine ou de facteur de croissance.

11. Conjugué protéinique magnétique selon la revendication 10, caractérisé en ce que P représente un anticorps monoclonal de la classe des IgG ou des IgM.

12. Conjugué protéinique magnétique selon la revendication 10, caractérisé en ce que P représente un anticorps monoclonal qui est dirigé contre un antigène qui est présent sous forme dissoute dans des solutions aqueuses de sels ou des liquides corporels.

13. Conjugué protéinique magnétique selon la revendication 10, caractérisé en ce que P représente un anticorps monoclonal qui est dirigé contre un antigène qui est exprimé sur des cellules, en particulier sur des cellules du système myéloïde ou lymphatique ou du sang périphérique, en particulier sur des lymphocytes B, des lymphocytes T ou leurs précurseurs ou sur des cellules tumorales, en particulier sur des cellules tumorales de la moelle osseuse.

14. Conjugué protéinique magnétique selon la revendication 13, caractérisé en ce que P représente un anticorps monoclonal qui est dirigé contre un antigène qui est exprimé sur des bactéries, des mycoplasmes ou des protozoaires, ou bien sur des virus.

15. Conjugué protéinique magnétique selon la revendication 1, caractérisé en ce que P représente un antigène.

16. Conjugué protéinique magnétique selon la revendication 1, caractérisé en ce que M est une particule dispersible comportant un noyau à base d'oxydes métalliques et une enveloppe portant des groupes amino, le noyau à base d'oxydes métalliques pouvant comprendre un groupe de substances paramagnétiques.

17. Conjugué protéinique magnétique selon la revendication 16, caractérisé en ce que le diamètre des particules est compris entre environ 0,1 et environ 100 µm, mais de préférence entre environ 0,1 et environ 1,5 µm.

18. Conjugué protéinique magnétique selon la revendication 1, caractérisé en ce que X peut être séparé par voie chimique ou enzymatique.

19. Procédé pour la préparation d'un conjugué protéinique magnétique de formule I, 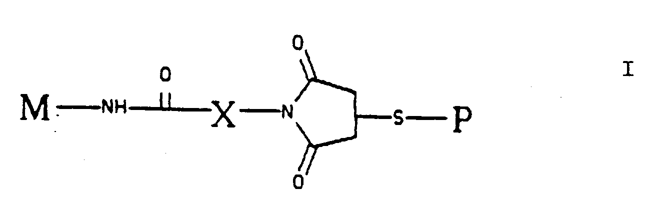 caractérisé en ce que l'on fait réagir une particule magnétique M portant des groupes amino avec un espaceur maléimido, réagissant avec des groupes amino, de formule générale II 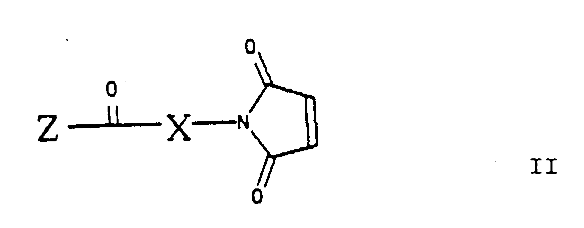 Z étant un groupe partant réactif, avec formation d'une liaison amide, pour aboutir à un composé de formule générale III 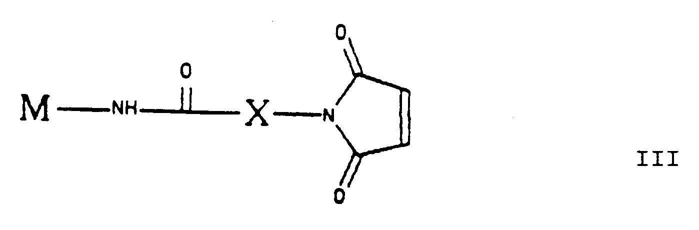 que l'on fait enfin réagir avec une protéine P portant des groupes mercapto, pour aboutir à un composé de formule générale I.

20. Procédé pour l'élimination d'un antigène, anticorps, récepteur, substrat, cofacteur ou d'un déterminant glucidique, dissous, à partir de solutions aqueuses de sels ou de liquides corporels, caractérisé en ce que l'on met à incuber la solution avec un conjugué protéinique magnétique approprié de formule I 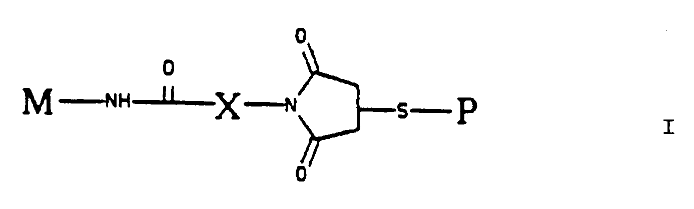 et on sépare par voie magnétique le conjugué protéinique magnétique, après adsorption spécifique du composant à éliminer, et éventuellement on sépare à nouveau par élution le composant spécifiquement adsorbé d'avec le conjugué protéinique magnétique.

21. Procédé pour l'élimination de cellules à partir de solutions aqueuses de sels ou de liquides corporels, caractérisé en ce que l'on met à incuber la suspension de cellules avec un conjugué protéinique magnétique approprié de formule I 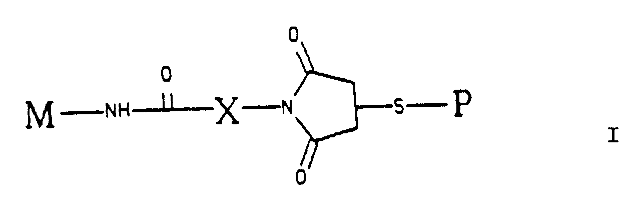 et on sépare par voie magnétique le conjugué protéinique magnétique, après adsorption spécifique des cellules à éliminer, et éventuellement on sépare à nouveau les cellules ou particules spécifiquement adsorbées d'avec le conjugué protéinique magnétique.

22. Utilisation d'un conjugué protéinique magnétique selon la revendication 1, pour l'élimination spécifique de cellules ou d'antigènes, récepteurs, substrats, cofacteurs ou déterminants glucidiques, solubles, à partir de solutions aqueuses de sels ou de liquides corporels, ou utilisation dans le cadre d'un procédé diagnostique ou en tant qu'agent de diagnostic.

23. Utilisation d'un conjugué protéinique magnétique selon la revendication 22, pour l'élimination de cellules selon la revendication 13 ou 14, de préférence pour la déplétion de moelle osseuse ou la typification de HLA.

## Revendications (Revendications pour l'(les) Etat(s) contractant(s) suivant(s): ES, GR)

1. Procédé pour la préparation d'un conjugué protéinique magnétique de formule générale I 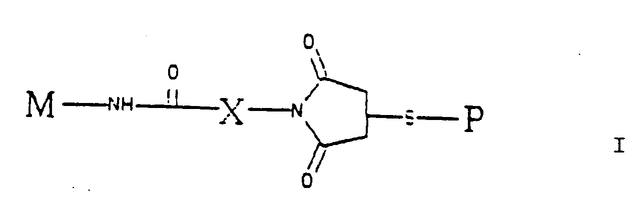 dans laquelle M est un matériau ou une particule dispersible, à réaction magnétique, qui porte des groupes amino, P est une protéine qui porte un ou plusieurs groupes mercapto, et X représente une structure chimique organique inerte qui relie de façon chimique les deux ligands,
caractérisé en ce que l'on fait réagir une particule magnétique M portant des groupes amino avec un espaceur maléimido, réagissant avec des groupes amino, de formule générale II 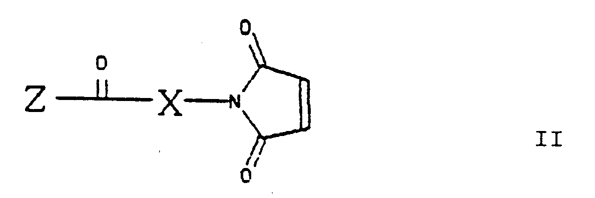 Z étant un groupe partant réactif, avec formation d'une liaison amide, pour aboutir à un composé de formule générale III 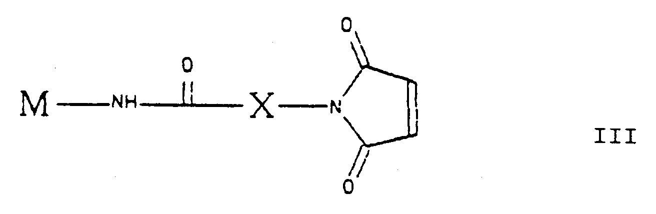 que l'on fait enfin réagir avec une protéine P portant des groupes thiol, pour aboutir à un composé de formule générale I.

2. Procédé pour la préparation d'un conjugué protéinique magnétique selon la revendication 1, caractérisé en ce que X est un espaceur aliphatique, aromatique, alicyclique, alicyclique-aliphatique ou aromatique-aliphatique, qui peut éventuellement être dans chaque cas substitué de façon appropriée.

3. Procédé pour la préparation d'un conjugué protéinique magnétique selon la revendication 2, caractérisé en ce que X est un groupe -(CH₂)ₙ- dans lequel n = 1 à 8, de préférence dans lequel n = 1 à 5 et en particulier dans lequel n = 2 ou 3, ou en ce que X est un groupe phénylène, les deux ligands pouvant être en position ortho, méta ou para, ou en ce que X est un groupe phénylène-(CH₂)ₙ- dans lequel n = 1 à 5, de préférence n = 3, le groupe phénylène étant relié au groupement succinimidyle, ou en ce que X est -CHR-, R représentant une chaîne latérale d'un aminoacide, de préférence la chaîne latérale de l'aminoacide alanine, sérine, thréonine ou méthionine, et en particulier la chaîne latérale de l'alanine, ou en ce que X est un groupe cyclohexylméthylène, le groupe méthylène étant relié au groupement succinimidyle, et, en ce qui concerne la fonction carbonyle reliée au cycle cyclohexyle, celle-ci étant de préférence liée en position 4 du cycle cyclohexyle, ou en ce que X est un groupe phénylène, le cycle phénylène étant toutefois substitué par un groupe méthyle, hydroxy, méthoxy, acétoxy, nitro ou cyano, ou par un atome de chlore ou de brome.

4. Procédé pour la préparation d'un conjugué protéinique magnétique selon la revendication 1, caractérisé en ce que les groupes thiol de la protéine P soit sont présents de façon naturelle, soit sont engendrés par réduction de liaisons disulfure, ou sont introduits dans la protéine par une réaction chimique.

5. Procédé pour la préparation d'un conjugué protéinique magnétique selon la revendication 1, caractérisé en ce que P peut être une immunoglobuline ou un reste d'immunoglobuline, de préférence un anticorps monoclonal ou un fragment Fab, Fab' ou F(ab')₂, un antigène ou un reste d'enzyme, d'hormone, de lectine ou de facteur de croissance.

6. Procédé pour la préparation d'un conjugué protéinique magnétique selon la revendication 5, caractérisé en ce que P est un anticorps monoclonal de la classe des IgG ou des IgM, de préférence un anticorps monoclonal qui est dirigé contre un antigène qui est présent sous forme dissoute dans des solutions aqueuses de sels ou des liquides corporels, ou est exprimé sur des cellules, en particulier sur des cellules du système myéloïde ou lymphatique ou du sang périphérique, en particulier sur des lymphocytes B, des lymphocytes T ou leurs précurseurs, ou sur des cellules tumorales, en particulier sur des cellules tumorales de la moelle osseuse, ou sur des bactéries, des mycoplasmes ou des protozoaires, ou bien sur des virus.

7. Procédé pour la préparation d'un conjugué protéinique magnétique selon la revendication 1, caractérisé en ce que P représente un antigène.

8. Procédé pour la préparation d'un conjugué protéinique magnétique selon la revendication 1, caractérisé en ce que M est une particule dispersible comportant un noyau à base d'oxydes métalliques et une enveloppe portant des groupes amino, le noyau à base d'oxydes métalliques pouvant comprendre un groupe de substances paramagnétiques, un diamètre de particules compris entre environ 0,1 et environ 100 µm étant préféré, et un diamètre de particules compris entre environ 0,1 et environ 1,5 µm étant particulièrement préféré.

9. Procédé pour la préparation d'un conjugué protéinique magnétique selon la revendication 1, caractérisé en ce que X peut être séparé par voie chimique ou enzymatique.

10. Procédé pour l'élimination d'un antigène, anticorps, récepteur, substrat, cofacteur ou d'un déterminant glucidique, dissous, à partir de solutions aqueuses de sels ou de liquides corporels, caractérisé en ce que l'on met à incuber la solution avec un conjugué protéinique magnétique approprié de formule I 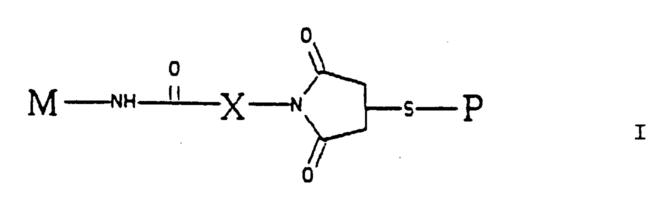 et on sépare par voie magnétique le conjugué protéinique magnétique, après adsorption spécifique du composant à éliminer, et éventuellement on sépare à nouveau par élution le composant spécifiquement adsorbé d'avec le conjugué protéinique magnétique.

11. Procédé pour l'élimination de cellules à partir de solutions aqueuses de sels ou de liquides corporels, caractérisé en ce que l'on met à incuber la suspension de cellules avec un conjugué protéinique magnétique approprié de formule I 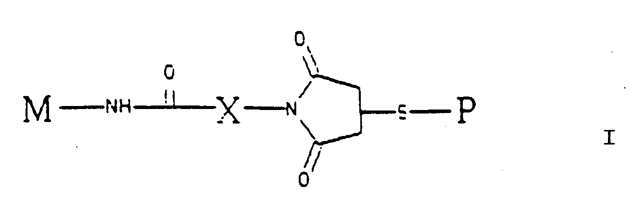 et on sépare par voie magnétique le conjugué protéinique magnétique, après adsorption spécifique des cellules à éliminer, et éventuellement on sépare à nouveau les cellules ou particules spécifiquement adsorbées d'avec le conjugué protéinique magnétique.

12. Utilisation d'un conjugué protéinique magnétique selon la revendication 1, pour l'élimination spécifique de cellules ou d'antigènes, récepteurs, substrats, cofacteurs ou déterminants glucidiques, solubles, à partir de solutions aqueuses de sels ou de liquides corporels, ou utilisation dans le cadre d'un procédé diagnostique ou en tant qu'agent de diagnostic.

13. Utilisation d'un conjugué protéinique magnétique selon la revendication 12, pour l'élimination de cellules selon la revendication 11, de préférence pour la déplétion de moelle osseuse ou la typification de HLA.
